# EUROPEAN PATENT SPECIFICATION

(11) **EP 2 160 139 B1**
(45) Date of publication and mention of the grant of the patent: **14.11.2012**
(21) Application number: 07747749.5
(22) Date of filing: 19.06.2007
(51) Int. Cl.: A61B 17/24, A61B 17/30, A61B 17/00, A61B 17/28, A61B 17/32

(54) **MULTIFUNCTIONAL SURGICAL INSTRUMENT FOR PRECISE OPERATIONS, PARTICULARLY FOR LARYNGOLOGIC AND NEUROSURGICAL OPERATIONS**
MULTIFUNKTIONALES OPERATIONSINSTRUMENT FÜR PRÄZISE OPERATIONEN, INSBESONDERE FÜR LARYNGOLOGISCHE UND NEUROCHIRURGISCHE OPERATIONEN
INSTRUMENT CHIRURGICAL MULTIFONCTIONNEL POUR OPÉRATIONS PRÉCISES, EN PARTICULIER LES OPÉRATIONS LARYNGOLOGIQUES ET NEUROCHIRURGICALES

(43) Date of publication of application: 10.03.2010
(73) Proprietor: Kukwa, Andrzej, 02-910 Warszawa (PL)
(72) Inventor: Kukwa, Andrzej, 02-910 Warszawa (PL)
(74) Representative: Grabowska, Malgorzata
(86) International application number: PCT/PL2007/000040
(87) International publication number: WO 2007/120063

(56) References cited:
- AU-B2- 513 224
- NL-A- 9 401 044
- US-A- 4 258 716
- US-A- 5 776 154
- US-A1- 2002 128 682
- US-A1- 2005 101 965

## Description

The present invention provides a multifunctional surgical instrument particularly for laryngologic and neurosurgical operations.

There are known surgical instruments in the form of tubes, inside of which a movable coaxial element is located, in particular cases in the form of a tube, cooperating with a head installed at one end of the instrument, said head being a working element and, depending on its design, performing different tasks, e.g. gripping, clamping, cutting, tissue breaking, etc. The other end of the instrument is connected to a handle, which at the same time drives the movable coaxial element.

Patent Application US 2005/0101965A1 discloses an apparatus and method for cutting and sealing blood vessel or tissue using a bipolar linear travel device. A handle is fixed in relation to the inner tube or outer tube. This is a permanent connection. The problem to be solved is regard as a provide a more lightweight construction.

US 4,258,716 discloses a microsurgical instrument which may be, for example, scissors or forceps. There is provided a tube with a rod and a handle with many working means for activate and manipulate the handle and a crank member.

Though the above-mentioned instruments accomplished in principle their tasks, they were generally designs which, due to their price, forced repeated use of the instrument as a whole, together with the head, since it was virtually undismountable.

WO 02/24084 discloses a surgical instrument for laryngologic operations, e.g. of the inferior turbinate, consisting of two coaxial tubes terminating at bladelike edges forming a cutting window. However, use of the instrument is limited by the rigid connection of the tubes.

There is a need in the field for a universal surgical instrument which due to the ease of manufacture and low production costs would allow to use it as a disposable device, leading to lower costs of sterilization and eliminating any hazards connected with imperfect functions of sterilizing equipment.

Therefore, it is an object of the present invention to provide a multifunctional surgical instrument particularly for laryngologic and neurosurgical operations.

These aims and others (presented below), which shall become more readily apparent in the description that follows, are achieved, in accordance with this invention with a multifunctional surgical instrument which has the structural and functional features described in the independent claim 1 herein, further embodiments of them being described in the dependent claims.

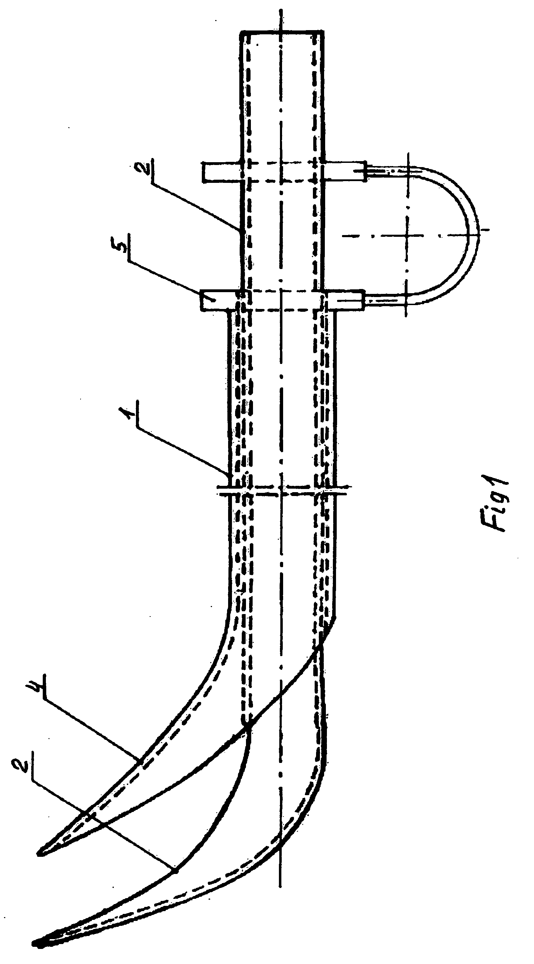

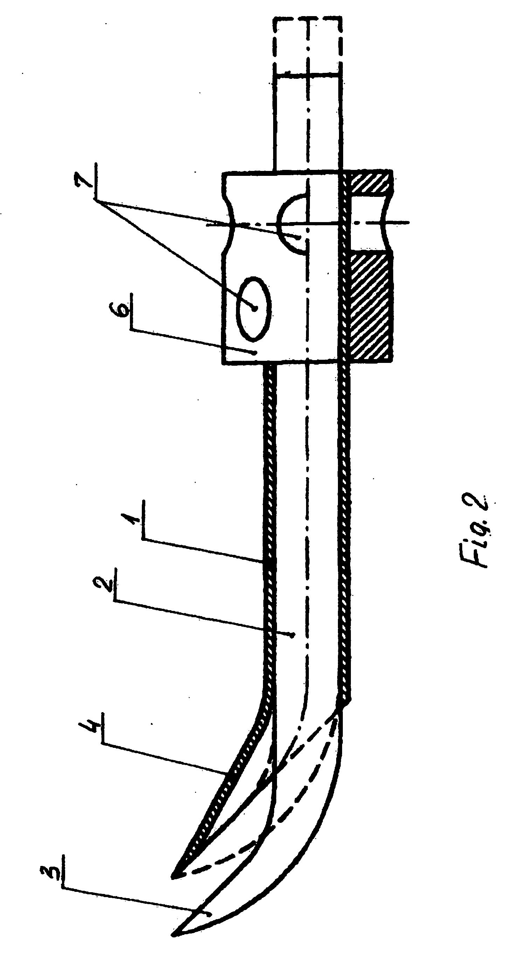

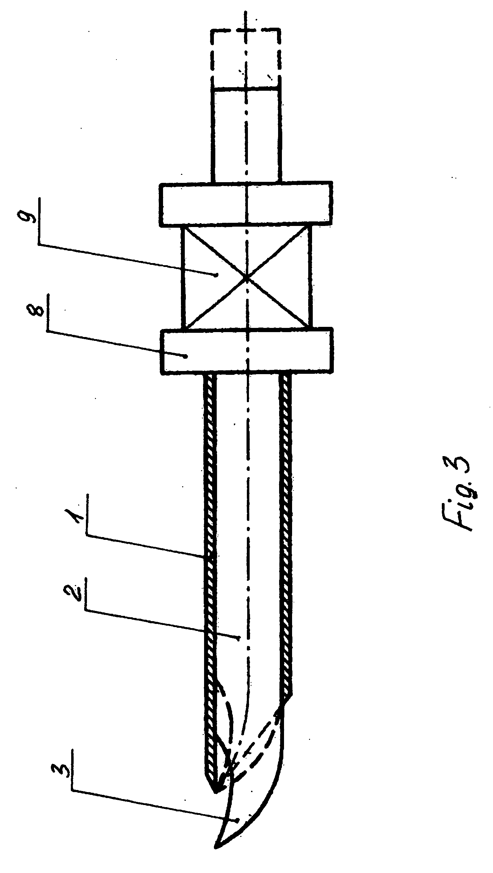

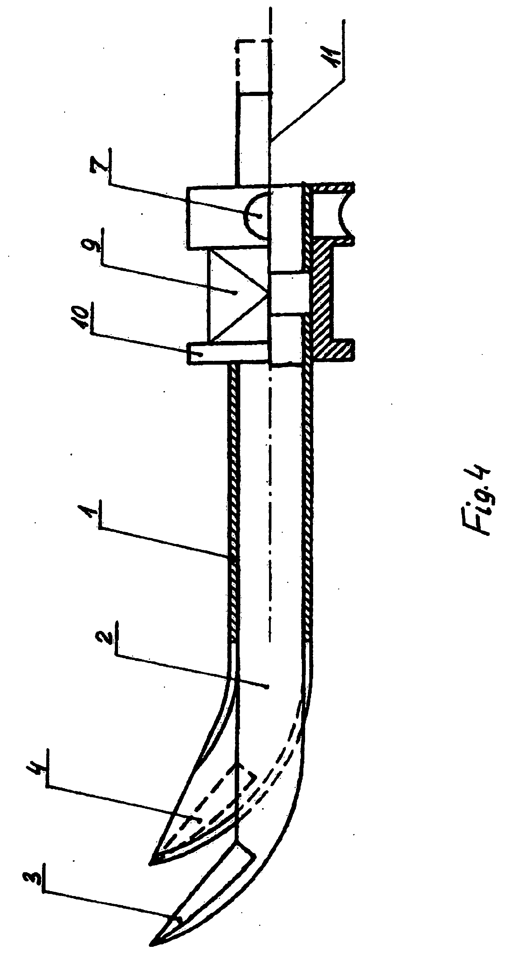

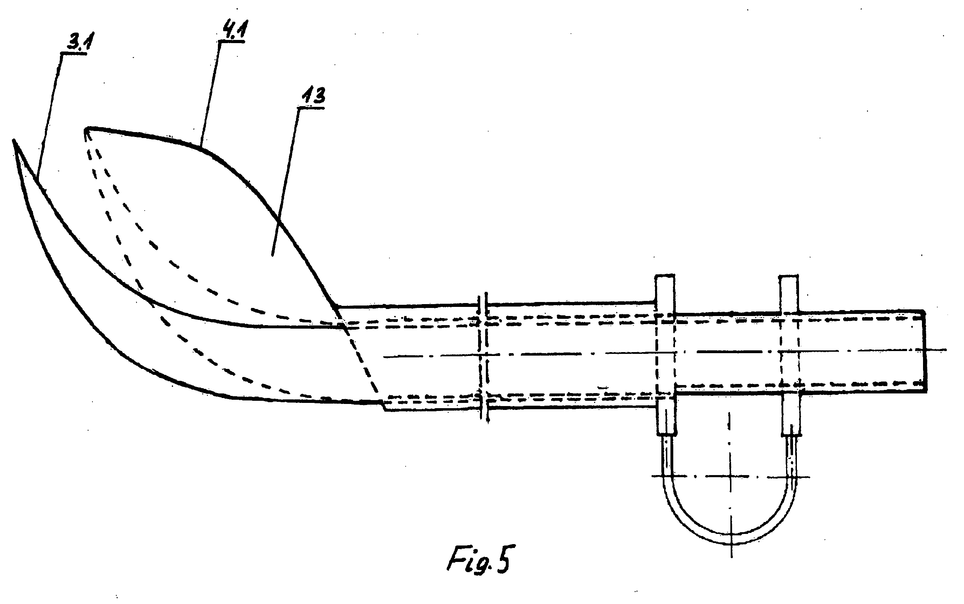

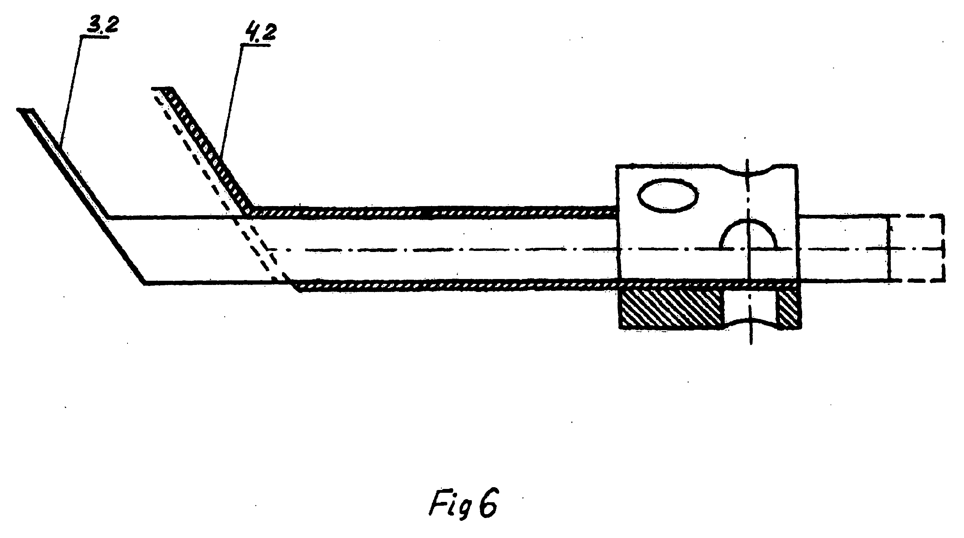

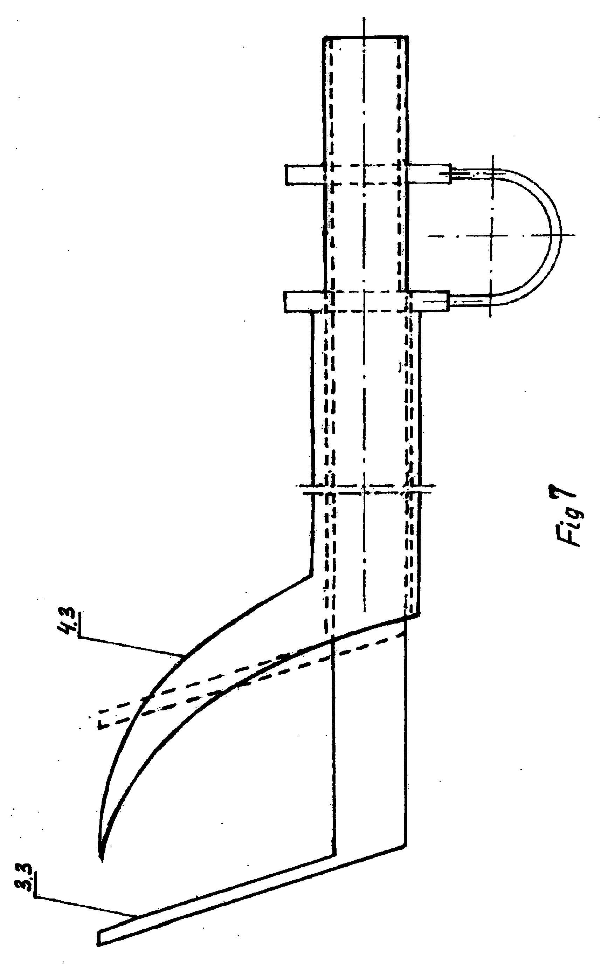

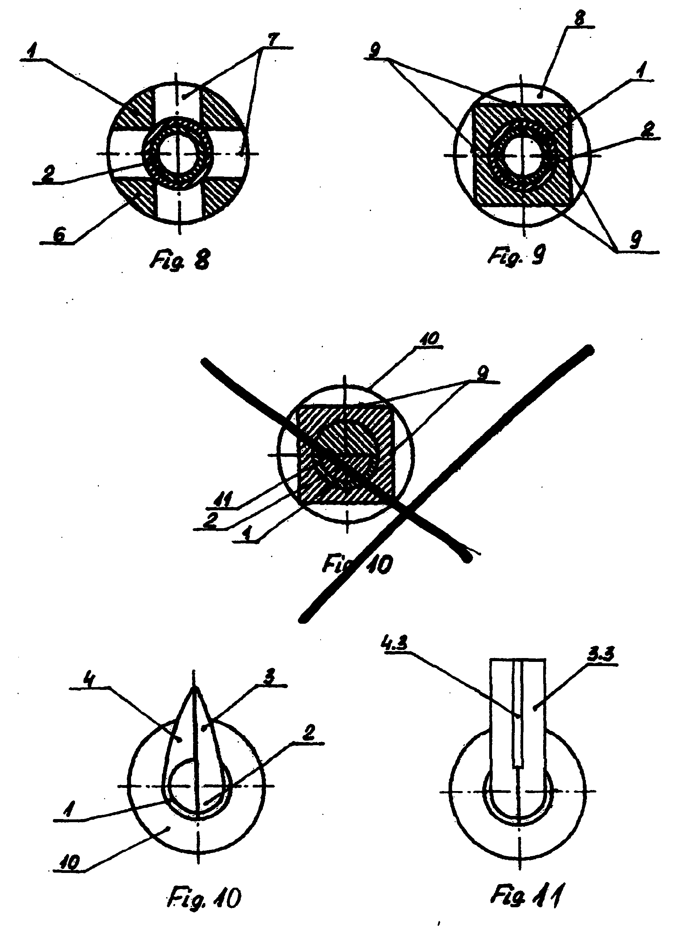

Surgical instruments are presented by the way of examples with reference to the accompany-ing drawings, in which: fig. 1 shows the side view of an instrument having both working ends arcuate; fig. 2 shows the instrument of fig. 1 with a fastening sleeve; fig. 3 shows an instrument similar to that of fig. 2, having only one arcuate working end; fig. 4 shows an instrument similar to that of fig. 1 and 2, but with another sleeve design; fig. 5 shows an instrument with the working ends with a clearance; fig. 6 shows an instrument with the spade-like working ends; fig. 7 shows an instrument according to the invention with one working end in the form of a flat spatula with a central lengthwise slit; fig. 8 shows the cross-sectional view of the sleeve from fig. 2; fig. 9 shows the cross-sectional view of the instrument of fig. 3; 4; fig. 10 shows the front view of the instrument of fig. 4; fig. 11 shows the working end side of the instrument of fig. 7.

The instrument consists of the external tube 1 with the working end 4, 4.1, 4.2, 4.3 and the internal coaxial tube 2 with the working end 3, 3.1, 3.2, 3.3 and the handle 5 of any known design providing a driving force for moving the internal tube and/or the external tube relative to each other, said handle being at the same time a handgrip. On shifting the internal coaxial tube 2 when the working ends 3, 3.1, 3.2, 3.3 and 4, 4.1, 4.2, 4.3 approach and press against each other, the grip effect is obtained.

The instrument shown in fig. 5 has the working end (4.1) of the external tube curved in a direction opposite to the curvature of the working end (3.2) of the internal coaxial tube to form a clearance in the form of an eye (13) when the working elements (3.1, 4.1) are pressed against each other.

The instrument shown in fig. 6 has the working ends (3.2, 4.2) in the form of flat spatulae both bent at the same angle relative to the instrument's axis.

The instrument according to the invention in the embodiment shown on fig. 7 has the working end (4.3) of the external tube curved with the convex edge directed to the handle, and the working end (3.3) of the internal coaxial tube bent relative the instrument's axis and in the form of a flat spatula with a central lengthwise slit.

In practice, the instrument according to the invention may have the fastening sleeve 6 or 8, or 10. Said sleeve may have holes 7 and/or chamfers 9, adapted to the handle design.

The microsurgical instrument according to the invention is easy and cheap to manufacture, it is advantageously versatile and, what has to be emphasized, its working parts are replaceable, which makes it possible to use disposable working parts, as in the case of syringe needles.

Continuous shortages of equipment and financial means for purchasing appropriate surgical instruments for different operation types resulted in the need for new solutions, and this problem was the basis for designing the novel instrument according to the invention.

The instrument according to the invention can be manufactured in various sizes (tube diameters) while maintaining crushing strength and deformation strength appropriate for surgical instruments.

Working parts of the instrument according to the invention could preferably be made of materials used in the manufacture of disposable needles. It seems that the majority of elaborated models could be made of known materials using known technologies.

Depending on the shape of working parts of the instrument said instrument could be used for different purposes, e.g. as forceps - so called flat forceps of Peano or Kocher surgical instrument type, or tweezers, holders, or even scissors. The distal - remote end would be the other end, so called working end, and would comprise an instrument in the form of a scalpel, small or larger hook, needle, separator - raspatory, or other surgical instrument. A different situation would arise in the case of endoscopy, where said end could house the optical system of the device.

A remarkable advantage of the instrument according to the invention is that it could be connected to an aspirator, allowing to remove e.g. tissue fragments detached from the body due to operation of working elements of the instrument.

## Claims

1. A multifunctional surgical instrument, particularly for laryngologic and neurosurgical operations, consisting of an external tube (1) and an internal element in a form of a tube (2) coaxial with the external tube, said external tube and internal tube having working ends, and a handle putting them into motion, the working ends (3, 4) of the external tube (1) and or internal coaxial tube (2) opposite to the handle are curved and optionally profiled, said external tube and internal element being coaxially slidable relative to each other, and said handle being separably mounted, **characterized in that** the working end (4.3) of the external tube is curved with the convex edge directed to the handle, and the working end (3.3) of the internal coaxial tube is bent relative the instrument's axis and has the form of a flat spatula with a central lengthwise slit.

2. The multifunctional instrument according to claim 1, **characterized in that** said working ends (3, 4) are arcuate in a curvature plane and chamfered in a common cylindrical and/or planar surface, which is perpendicular to the curvature plane.

3. The multifunctional instrument according to claim 1, **characterized in that** the external tube (1) is fitted with an attachment in a form of a sleeve (6) with holes (7), being an element fastening the handle.

## Patentansprüche

1. Multifunktionales chirurgisches Instrument, insbesondere für laryngologische und neurochirurgische Operationen, bestehend aus einem Außenrohr (1) und einem inneren Element in Form einer koaxial zum äußeren Rohr angeordneten Rohr (2), wobei das Außenrohr und das Innenrohr mit Arbeitsenden und einem Handgriff, der sie in Bewegung bringt, versehen sind, die zu dem Handgriff entgegengesetzten Arbeitsenden (3, 4) des Außenrohrs (1) und/oder des koaxialen Innenrohrs (2) gebogen und gegebenenfalls profiliert sind, wobei das Außenrohr und das innere Element koaxial zueinander verschiebbar sind, und wobei der Handgriff lösbar angeordnet ist, **dadurch gekennzeichnet, dass** das Arbeitsende (4.3) des Außenrohrs mit der konvexen Kante auf den Handgriff gerichtet gekrümmt ist, und das Arbeitsende (3.3) des koaxialen Innenrohrs zur Instrumentenachse gebogen ist und die Form eines flachen Spatel mit einem zentralen Längsschlitz hat.

2. Multifunktionales Instrument nach Anspruch 1, **dadurch gekennzeichnet, dass** das die Arbeitsenden (3, 4) in einer Krümmungsebene gekrümmt und abgeschrägt in einer gemeinsamen zylindrischen und/oder flachen Oberfläche sind, welche senkrecht zu der Krümmungsebene liegt.

3. Multifunktionales Instrument nach Anspruch 1, **dadurch gekennzeichnet, dass** das Außenrohr (1) mit einem Aufsatz in Form einer Hülse (6) mit Löchern (7) ausgestattet ist, welches ein Element zur Befestigung des Handgriffs bildet.

## Revendications

1. Instrument multifonctionnel de chirurgie, particulièrement pour des opérations e laryngologie et de neurochirurgie, constitué d'un tube (1) externe et d'un élément interne en forme de tube (2) coaxial avec le tube externe, ledit tube externe et le tube interne ayant des extrémités d'attaque, et une poignée les mettant en mouvement, lest extrémités d'attaque (3, 4) du tube (1) externe et/ou du tube (2) interne coaxial opposés à la poignée étant recourbées et optionnellement profilées, lesdit tube externe et element interne pouvant glisser coaxialement l'un relativement à l'autre, ladite poignée étant monté séparément, **caractérisé en ce que** l'extrémité d'attaque (4.3) du tube externe est recourbée avec son bord convexe orienté vers la poignée, et l'extrémité d'attaque (3.3) du tube interne coaxial est recourbée relativement à l'axe de l'instrument et a la forme d'une spatule plate avec une fente centrale longitudinale.

2. Instrument multifonctionnel selon la revendication 1, **caractérisé en ce que** lesdites extrémités d'attaque (3, 4) sont arquées dans un plan de courbure et chanfreinées en forme d'une surface commune cylindrique et/ou plane, qui est perpendiculaire au plan de courbure.

3. Instrument multifonctionnel selon la revendication 1, **caractérisé en ce que** le tube (1) externe est muni d'un accessoire en forme de douille (6) avec des trous (7), ladite douille étant un élément fixant ladite poignée.
